# EUROPEAN PATENT APPLICATION

(11) **EP 4 019 630 A1**
(43) Date of publication of application: **29.06.2022**
(21) Application number: 21217109.4
(22) Date of filing: 22.12.2021
(51) Int. Cl.: C12N 9/24, C07K 14/32

(54) **FUCOIDAN DEGRADING ENZYME-PRODUCING GENETIC ENGINEERED STRAIN AND PREPARATION METHOD THEREOF**

(30) Priority: 25.12.2020 CN 202011558474
(71) Applicant: Beijing Leili Marine Bioindustry Inc., Beijing (CN)
(72) Inventor: Tang, Jie, Haidian District, Beijing (CN); Yan, Guofu, Haidian District, Beijing (CN)
(74) Representative: Geskes, Christoph

(57) **Abstract**

The invention relates to the field of microbial fermentation, in particular to a fucoidan degrading enzyme-producing genetic engineered strain and a preparation method thereof. The sequence of the 16S rDNA transferred into the fucoidan degrading enzyme-producing genetic engineered strain is shown as SEQ ID NO:1. The strain of the invention has the advantages of fast growth, high enzyme activity, good enzyme stability, strong adaptability to substrates from different sources, etc., and therefore has good application prospects.

## Description

### FIELD OF THIS APPLICATION

The present invention relates to the field of microbial fermentation, in particular to a fucoidan degrading enzyme-producing genetic engineered strain and a preparation method thereof.

### BACKGROUND

Fucoidan is a high-molecular-weight sulfated heteropolysaccharide widely found in rockweed, mainly composed of L-fucose and sulfate groups; in addition, it also contains galactose, Xylose, glucose, mannose, rhamnose, and uronic acid etc. Studies have found that fucoidan has a variety of physiological activities, including anticoagulant, anti-tumor, antiinflammatory, anti-thrombotic, immunity enhancement, and anti-infection etc. Therefore, it has become one of the main study directions of natural marine active substances today. Because the molecular mass of fucoidan is huge, such as ranging from tens of thousands to hundreds of thousands, it disadvantageously affects its absorption efficiency; at the same time, fucoidan usually has a certain viscosity, which greatly hinders its use as a medicine. The degradation of large-molecular-weight fucoidan into small-molecular-weight oligosaccharides can not only solve the above-mentioned application problems, but also enhance its physiological activity in some cases. While, Fucoidan being transformed into small molecular weight oligosaccharides is also an important method for structure-activity research. Therefore, whether from the perspective of structure research or the application perspective of its activity, the degradation of fucoidan is very important.

Currently, the methods for degrading Fucoidan mainly include chemical degradation, physical degradation and enzymatic degradation. The chemical degradation method is nonspecific degradation, the reaction conditions are harsh, the hydrolysis is difficult to control, and the product conversion rate is low, and the functionality is low. Although the physical degradation method is simple to operate and has good controllability, its degradation efficiency is generally low, and it is difficult to obtain products that meet a certain molecular weight range. The best method is enzymatic hydrolysis, which can specifically and selectively cut the glycosidic bonds on the fucoidan chain, the reaction process is easy to control, and it is easy to obtain oligosaccharide products with the required molecular weight range, and the product conversion rate is high and functionality is high. At present, there have been some reports on the research of fucoidan at home and abroad. Bakunina et al. screened a enzyme-producing strain from rockweed, and its enzyme activity can reach 1920U; Sakai et al. screened a enzyme-producing strain from seawater, which was identified by 16s rRNA as belonging to Alteromonadaceae; Kim et al. screened a enzyme-producing strain from rockweed, which was identified as belonging to Sphingomonas, and the produced enzyme is endonuclease.

In short, there is no commercial enzyme for the fucoidan at present, because the enzyme-producing capacity of these microorganisms has not yet reached the requirements of commercialization. Therefore, improving the activity of fermentation enzymes has become the focus of the current research field. For example, Zhao Likun et al, College of Life Sciences, Hebei University used ultraviolet-lithium chloride (UV-LiCI) and ultraviolet-nitro-soguanidine (UV-NTG) to perform compound mutagenesis on original strains, and obtained a high-yielding strain with a relative increase of 65% of enzyme. In Biotechnology and Biopharmaceutical Laboratory of Shenyang Pharmaceutical University, NTG, DES, ultraviolet rays and other mutagenic factors were used to perform multiple mutagenesis treatments on the original strains of fucoidan degrading strain, and the output has been significantly improved. Chinese patent application CN201110003059.2 discloses a fucoidan-degrading enzyme producing strain and a preparation method thereof, in which through repeated mutagenesis and screening, a high-yield strain named FT26-9 was obtained, and it can increase the fermentation titer by about 40%. However, since most of the above studies used highly toxic organic reagents with carcinogenic effects, and the mutagenesis process is complex, which has an adverse effect on human health and environmental protection, the above technical solutions need to be further improved.

### SUMMARY OF THE INVENTION

In view of this, the present invention provides a fucoidan degrading enzyme-producing genetic engineered strain and a preparation method thereof. The strain has the advantages of fast growth, high enzyme activity, good enzyme stability, and strong adaptability to different substrates.

In order to achieve the above-mentioned purpose of the invention, the present invention provides the following technical solutions:

According to one aspect, there is provided 16s rDNA, the sequence of which is shown in SEQ ID NO:1.

According to another aspect, there is provided application of 16S rDNA in the preparation of a fucoidan degrading enzyme-producing genetic engineered strain.

According to yet another aspect, there is provided a plasmid, which has the above-mentioned 16S rDNA.

In one embodiment, the vector of the plasmid is a PSKH plasmid.

According to yet another aspect, there is provided a genetically engineered strain, which comprises the above-mentioned 16S rDNA, or the above-mentioned plasmid.

According to yet another aspect, there is provided a fermentation method of the genetically engineered strain, including the following steps:
(1) Plate culturing: inoculating the strain on a plate medium, the culturing temperature is 26-30 °C, and the culturing time is 6 ^{~}7d;
(2) Slant culturing: inoculating the strain cultured in step (1) on a slant medium, the culturing temperature is 26-30 °C, and the culturing time is 6-7d;
(3) Seed culturing: inoculating the strain cultured in step (2) into a seed medium, the liquid volume is 8vt%-20vt%, the culturing temperature is 26-30 °C, and the rotation speed is 180-220rpm, and the culturing time is 30-45h;
(4) Fermentation culturing: inoculating the seed culture solution in step (3) into the fermentation medium, the inoculum is 2%-10% (v/v), the liquid volume is 14vt%-26vt%, the culturing temperature is 26-30 °C, and culturing for 8-10 days at180-240rpm rotation speed.

Preferably, the plate medium contains the following components in mass percentage: carbon source 0.5% to 1.5%, nitrogen source 0.1% to 0.1%. 3%, inorganic salt 0.5% to 1.0%, agar 1.2% to 1.8%, the rest is water, pH is 7.2 to 7.4;

In the plate medium, the carbon source is one of or a mixture of glucose and soluble starches; the nitrogen source is one of or a mixture of ammonium acetate and ammonium sulfate; the inorganic salt is one of or a mixture of two or more of sodium salt, potassium salt, magnesium salt, and calcium salt.

Preferably, the slant medium contains the following components in mass percentage: carbon source 0.5% to 1.5%, nitrogen source 0.1% to 0.1%. 3%, inorganic salt 0.5% to 1.0%, agar 1.2% to 1.8%, the rest is water, pH is 7.2 to 7.4;

In the slant medium, the carbon source is one of or a mixture of glucose and soluble starches; the nitrogen source is one of or a mixture of ammonium acetate and ammonium sulfate; the inorganic salt is one of or a mixture of two or more of sodium salt, potassium salt, magnesium salt, and calcium salt

Preferably, the seed culture medium contains the following components in mass percentage: carbon source 2.0% to 3.0%, nitrogen source 2.0% to 4.0%, and trace elements 0.0001% to0.0004%, amylase 3^{~}5u/g starch, the rest is water, pH is 7.0^{~}7.2;

In the seed culture medium, the carbon source is one of or a mixture of corn starch and glucose; the nitrogen source is one of or a mixture of two or more of peanut meal, soybean meal, corn steep liquor, yeast powder and yeast extract; and the trace element is cobalt chloride.

Preferably, the fermentation medium contains the following components in mass percentage: carbon source 5.0% to 15.0%, nitrogen source 2.0%^{~}4.0%, trace element 0.0001%^{~}0.0004%, amylase 3^{~}5u/g starch, the rest is water, pH is 7.0^{~}7.2;

In the fermentation medium, the carbon source is one of or a mixture of two or more of corn starch, glucose and maltose; the nitrogen source is one of or a mixture of two or more of peanut meal, soybean meal, corn steep liquor and yeast powder; the trace element is cobalt chloride.

The present invention provides a fucoidan degrading enzyme-producing genetic engineered strain and a preparation method thereof. The sequence of the 16s rDNA is shown in SEQ ID NO:1. The beneficial effects of the present invention are as follows:

The present invention used the combination of low-energy nitrogen ion implantation-lithium chloride composite mutagenesis and ultraviolet-lithium chloride composite mutagenesis, and finally screened and obtained a fucoidan degrading enzyme-producing strain. The activity of the fucoidan degrading enzyme in the fermentation product was greatly improved, and the genetic stability was good. The enzyme activity did not change significantly after 5 passage times. In a 500 ml fermentation flask, using glucose as the quick-acting carbon source and corn starch as the slow-acting carbon source, the enzyme activity of the fucoidan-degrading enzyme produced by fermentation reached 304 U/mL, which was 23.4% higher than that of the original strain. The present invention fully meets the mutagenesis and screening requirements of industrialization of foucoidan degrading enzyme-producing strains, can be applied to industrialized fermentation production, and has great social significance and economic value.

The present invention used the fusion PCR technology to repeatedly clone the 16S DNA fragment, and found that the enzyme activity was doubled, which proved that the gene fragment is an active fragment and plays a key role.

The fermentation medium of the strain of the present invention has simple ingredients, which are very common and are commercially available easily, and the fermentation effect is significant, which can greatly reduces the production cost of fucoidan degrading enzymes, can be applied to large-scale industrial production of Fucoidan degrading enzyme, and has great economic application value.

### DETAILED DESCRIPTION OF THE INVENTION

The invention discloses a genetically fucoidan degrading enzyme-producing engineered strain and a preparation method thereof. Those skilled in the art can learn from the content of this application and appropriately improve the process parameters. In particular, it should be pointed out that all similar substitutions and modifications are obvious to those skilled in the art, and they are all considered to be included in the present invention. The method and application of the present invention have been described through the preferred embodiments. It is obvious that one skilled in the art can make modifications or appropriate changes and combinations to the methods and applications described herein without departing from the content, spirit and scope of the present invention, which will fall within the scope of the present invention.

The materials and reagents involved in the following examples for preparing the fucoidan degrading enzyme-producing genetic engineered strain are commercially available.

The present invention will be further explained below in conjunction with examples:

### Example 1:

This example illustrates a method for mutagenesis of fucoidan-degrading strain as original strain.

The fucoidan-degrading strain FE001 preserved in the laboratory was used as original strain, the mutagenesis was carried out, and the specific steps were as follows:
(a) Preparation of single spore suspension: the fresh slant of fucoidan-degrading strain which has been cultured for 6^{~}7d at 26^{~}30 °C constant temperature was taken and 10mL of sterile water added to which, the spores was scraped and poured into a 250mL Erlenmeyer flask with certain glass beads, and the flask was shaken well for 20min (at 250rpm) to disperse the spore chains; then the solution was filtered with three layers of degreasing gauze, and the number of the spores in the filtrate was counted through a hemocytometer and the spore concentration was adjusted to 10⁶/ml.
(b) low-energy nitrogen ion implantation-lithium chloride compound mutagenesis: 0.1 mL the spore suspension of step (a) was taken and evenly coated on several sterile empty plates, and the plates were observed under microscope and if no cell overlaps, performing low-energy Nitrogen ion implantation. The low-energy nitrogen ion implanter in this experiment is a multi-function implanter. The fucoidan-degrading strains were implanted with ions under the energy of 10KeV, 14KeV, 16KeV, and 18KeV, respectively. The implantation doses were 40×10¹⁴ions/cm², 80×10¹⁴ions/cm², 120×10¹⁴ions/cm², 160×10¹⁴ions/cm², 200×10¹⁴ions/cm², and 240×10¹⁴ions/cm². The vacuum of the target chamber is 10⁻³ Pa, and the implantation was pulsed at 20S with an interval of 15s. A control sample that does not accept implantation is placed in the target chamber. It was determined that the mutagenic energy being 16KeV and the mutagenic dose being 160×10¹⁴ions/cm²was the best low-energy nitrogen ion implantation mutagenesis conditions. After the ion implantation, the plate was taken out, eluted with 1ml of sterile water in a sterile environment, and the strains were coated on a lithium chloride plate medium, and cultured at 26-30 °C for 6-7 days by inverting the plate.
(c) UV-lithium chloride compound mutagenesis: 5 mL of the spore suspension in step (a) was taken and transferred into several sterile empty plates containing a magnetic rotor respectively, and the plated were placed on a magnetic stirrer. The 20W UV lamp was turned onto preheat for 20 minutes, then the height of the plates were adjusted at 30cm under the UV lamp, the lid of the plates were opened, and irradiate them for 30s, 45s, 60s, 75s, and 90s respectively. After the mutagenesis, the bacterial suspension was applied to the plates with lithium chloride medium, and cultured for 6 to 7 days in the inverted and darkened conditions at a constant temperature of 26-30 °C. It was determined that 60s was the best time for UV mutagenesis, and the positive mutation rate was the highest at this time, reaching 18.1%.
(d) Screening of mutant strains:

Initial Screening: the single colonies mutagenized in step (b) and step (c) ware inoculated to a slant medium and cultured at 26-30 °C. After 6^{~}7d, one-half of the colonies was shoveled and transferred into a centrifuge tube, and was soaked in 0.5 mL of acetone for 12-15 hours, then 0.25 mL of ethyl acetate was added and being mixed quickly, and then was centrifuged at 3000 rpm for 10 minutes; The supernatant was taken to determine the fucoidan-degrading enzyme B1a titer in the extract of each strain, and 50% of the high-titer strains were selected for fermentation in a shake flask for further screening.

Further screening: the strains selected by the initial screening were cultured on a slant, and then was inoculated into the seed medium for further culturing for 30-45 hours at 26-30 °C and 180-220 rpm shaker rotation speed; and then was inoculated into the fermentation medium with 2-10% (v/ v) of inoculum volume, and cultured at 26^{~}30 °C, 180^{~}240rpm shaker speed for 8^{~}10 d; After this, 3mL fermentation broth was taken and transferred into a centrifuge tube, and centrifuged at 3500rpm for 10min; the supernatant was discarded from the centrifuge tube, methanol was added to the centrifuge tube to reach 9mL, and then the centrifuge tube was shaken on a fast mixer for 30 minutes, and centrifuged at 3500 rpm for 10 minutes; The supernatant was taken to determine the fucoidan-degrading enzyme B1a titer in the fermentation broth. The strain with the highest titer of the fucoidan degrading enzyme B1a in the fermentation broth was selected as the original strain for the next mutagenesis, so as to repeat steps (a) to (d), as shown below:
(a) Preparation of single spore suspension: the fresh slant of fucoidan-degrading strain which has been cultured for 6^{~}7d at 26^{~}30 °C constant temperature was taken and 10mL of sterile water added to which, the spores was scraped and poured into a 250mL Erlenmeyer flask with certain glass beads, and the flask was shaken well for 20min (at 250rpm) to disperse the spore chains; then the solution was filtered with three layers of degreasing gauze, and the number of the spores in the filtrate was counted through a hemocytometer and the spore concentration was adjusted to 10⁶/ml.
(b) low-energy nitrogen ion implantation-lithium chloride compound mutagenesis: 0.1 mL the spore suspension of step (a) was taken and evenly coated on several sterile empty plates, and the plates were observed under microscope and if no cell overlaps, performing low-energyNitrogen ion implantation. The low-energy nitrogen ion implanter in this experiment is a multi-function implanter. The fucoidan-degrading strains were implanted with ions under the energy of 10KeV, 14KeV, 16KeV, and 18KeV, respectively. The implantation doses were 40×10¹⁴ions/cm², 80×10¹⁴ions/cm², 120×10¹⁴ions/cm², 160×10¹⁴ions/cm², 200×10¹⁴ions/cm², and 240×10¹⁴ions/cm². The vacuum of the target chamber is 10⁻³ Pa, and the implantation was pulsed at 20S with an interval of 15s. A control sample that does not accept implantation is placed in the target chamber. It was determined that the mutagenic energy being 16KeV and the mutagenic dose being 160×10¹⁴ions/cm²was the best low-energy nitrogen ion implantation mutagenesis conditions. After the ion implantation, the plate was taken out, eluted with 1ml of sterile water in a sterile environment, and the strains were coated on a lithium chloride plate medium, and cultured at 26-30°C for 6-7 days by inverting the plate.
(c) UV-lithium chloride compound mutagenesis: 5 mL of the spore suspension in step (a) was taken and transferred into several sterile empty plates containing a magnetic rotor respectively, and the plated were placed on a magnetic stirrer. The 20W UV lamp was turned onto preheat for 20 minutes, then the height of the plates were adjusted at 30cm under the UV lamp, the lid of the plates were opened, and irradiate them for 30s, 45s, 60s, 75s, and 90s respectively. After the mutagenesis, the bacterial suspension was applied to the plates with lithium chloride medium, and cultured for 6 to 7 days in the inverted and darkened conditions at a constant temperature of 26-30 °C. It was determined that 60s was the best time for UV mutagenesis.
(d) Screening of mutant strains:

Initial Screening: the single colonies mutagenized in step (b) and step (c) ware inoculated to a slant medium and cultured at 26-30 °C. After 6^{~}7d, one-half of the colonies was shoveled and transferred into a centrifuge tube, and was soaked in 0.5 mL of acetone for 12-15 hours, then 0.25 mL of ethyl acetate was added and being mixed quickly, and then was centrifuged at 3000 rpm for 10 minutes; The supernatant was taken to determine the fucoidan-degrading enzyme B1a titer in the extract of each strain, and 50% of the high-titer strains were selected for fermentation in a shake flask for further screening.

Finally, the target strain streptomyces avermitilis AVE07-N2-16515 with high production of fucoidan degrading enzyme B1a was screened out.

The morphological and physiological and biochemical characteristics of the strain of the present invention are as follows:
Colony color: off-white.
Aerobic mode: aerobic growth.
Colony size: 2^{~}5mm.
Suitable growth temperature: 27.5 to 28.5 °C.
Suitable growth pH: 7.2 to 7.4.
Cell morphology: round colony, lusterless
Gram stain: positive.
The 16S rDNA sequence is:

GCAGCTACAGATAGACATGATCGTCGAGCAGAGTGGATTAAGAGCTG TGCTCTTATGAAGTTAGCGG
CGGACGGGTGAGTAACACGTGGGTAAC CTGCCCATAAGACTGGGATAACTCCGGGAAACCGGGGCTAATACCGG
ATAACATTTTGAACCGCATGGTTCGAAATTGAAAGGCGGCTTCGGCTG TCACTTATGGATGGACCCGCGTCGCAT
TAGCTAGTTGGTGAGGTAACG GCTCACCAAGGCAACGATGCGTAGCCGACCTGAGAGGGTGATCGGCC ACACTG
GGACTGAGACACGGCCCAGACTCCTACGGGAGGCAGCAGTA GGGAATCTTCCGCAATGGACGAAAGTCTGACGGA
GCAACGCCGCGTG AGTGATGAAGGCTTTCGGGTCGTAAAACTCTGTTGTTAGGGAAGAAC AAGTGCTAGTTGAA
TAAGCTGGCACCTTGACGGTACCTAACCAGAAA GCCACGGCTAACTACGTGCCAGCAGCCGCGGTAATACGTAGG
TGGCA AGCGTTATCCGGAATTATTGGGCGTAAAGCGCGCGCAGGTGGTTTCTT AAGTCTGATGTGAAAGCCCAC
GGCTCAACCGTGGAGGGTCATTGGAA ACTGGAGACTTGAGTGCAGAAGAGGAAAGTGGAATTCCATGTGTAG C
GGTGAAATGCGTAGAGATATGGAGGAACACCAGTGGCGAAGGCGA CTTTCTGGTCTGTAACTGACACTGAGGCGC
GAAAGCGTGGGGAGCAA ACAGGATTAGATACCCTGGTAGTCCACGCCGTAAACGATGAGTGCTA AGTGTTAGAG
GGTTTCCGCCCTTTAGTGCTGAAGTTAACGCATTAAGC ACTCCGCCTGGGGAGTACGGCCGCAAGGCTGAAACTC
AAAGGAATTG ACGGGGGCCCGCACAAGCGGTGGAGCATGTGGTTTAATTCGAAGCAA CGCGAAGAACCTTACCA
GGTCTTGACATCCTTTGACAACCCTAGAGAT AGGGCTTCTCCTTCGGGAGCAGAGTGACAGGTGGTGCATGGTTG
TCGT CAGCTCGTGTCGTGAGATGTTGGGTTAAGTCCCGCAACGAGCGCAAC CCTTGATTTTAGTTGCCATCATT
TAGTTGGGCACTTTAAGGTGACTGC CGGTGACAAACCGGAGGAAGGTGGGGATGACGTCAAATCATCATGCC CC
TTATGACCTGGGCTACACACGTGCTACAATGGACGGTACAAAGAG CTGCAAGACCGCGAGGTGGAGCTAATCTCA
TAAAACCGTTCTCAGTT CGGATTGTAGGCTGCAACTCGCCTACATGAAGCTGGAATCGCTAGTA ATCGCGGATC
AGCATGCCGCGGTGAATACGTTCCCGGGCCTTGTACA CACCGCCCGTCACACCACGAGAGTTTGTAACACCCGAGA
AGTCGGTGG GGTAACCAAGTGGAGCCAACGTACCTAAAGAGCTGCG。

The lithium chloride plate medium used in step (b) and step (c) comprises: soluble starch 1.0%, (NH₄)₂SO₄ 0.25%, NaCl 0.05%, MgSO₄·7H₂O 0.12%, K₂HPO₄·3H₂O 0.3%, CaCO₃ 0.3%, lithium chloride 1.0 mol·L⁻¹, agar powder 1.5%, the rest is distilled water, pH is 7.2 ^{~} 7.4.

The slope medium used in steps (a) and (d) comprises: soluble starch 1.0%, (NH₄)₂SO₄ 0.25%, NaCl 0.05%, MgSO₄·7H₂O 0.12%, K₂HPO₄ ·3H₂O 0.3%, CaCO₃ 0.3%, agar powder 1.5%, the rest is distilled water, and pH is 7.2 ^{~} 7.4.

The seed medium used in step (d) is: corn starch 1.5%, glucose 1.0%, peanut meal 1.0%, soybean meal 0.8%, yeast powder 0.5%, CoCl₂·6H₂O 0.0003%, amylase 4u/g starch, pH 7.0 ^{~} 7.2, prepared with tap water.

The fermentation medium used in step (d) is: corn starch 6%, glucose 2%, peanut meal powder 1.4%, soybean meal powder 1.2%, yeast powder 0.3%, CoCl2·6H2O 0.0003%, Amylase 4u/g starch, the rest is distilled water, and pH is 7.0 ^{~} 7.2.

### Example 2:

This example illustrates the passage stability of the mutant strain.

In the fermentation medium with glucose as the quick-acting carbon source and corn starch as the slow-acting carbon source, the passage stability of the mutant strain AVE07-N2-16515 was tested, and the results of the strain passage fermentation test are shown in Table 1:

**Table 1 High-yielding mutant strain AVE07-N2-16515 passage stability test [0065]**

| generations | Sampling volume ml | starting strain | Solution for inoculation | Volume ml | inoculated substances and concentrations | Culturing Time h | Enzyme activity U/mL |
|---|---|---|---|---|---|---|---|
| First | 1 | The mutant strain from example 1 | domestication and enrichment nutrient solution for first generation | 200 | Fucoidan 2g/L | 48 | 230 |
| Second | 5 | Fermentation broth of first generation | domestication and enrichment nutrient solution for second generation | 200 | Fucoidan 4g/L | 48 | 250 |
| Third | 5 | Fermentation broth of second generation | domestication and enrichment nutrient solution for third generation | 200 | Fucoidan 6g/L | 48 | 260 |
| Forth | 5 | Fermentation broth of third generation | domestication and enrichment nutrient solution for forth generation | 200 | Fucoidan 8g/L | 48 | 280 |
| Fifth | 5 | Fermentation broth of forth generation | domestication and enrichment nutrient solution for fifth generation | 200 | Fucoidan 10g/L | 48 | 300 |

From the experimental results, it can be seen that after 5 generations, the fucoidan degrading enzyme B1a titer of the mutant strain AVE07-N2-16515 is relatively stable, it means that the mutant strain has good passage stability, and can be used as a production strain for further research and development.

### Example 3:

This example illustrates the process for producing fucoidan-degrading enzyme by fermentation of the fucoidan-degrading enzyme-producing strain AVE07-N2-16515.

The composition of the medium used in this example:
Plate medium: soluble starch 1.0%, (NH₄)₂SO₄ 0.25%, NaCl 0.05%, MgSO₄·7H₂O 0.12%, K₂HPO₄·3H₂O 0.3%, CaCO₃ 0.3%, agar powder 1.5%, the rest is distilled water and pH is 7.2 to 7.4.
Slant medium: soluble starch 1.0%, (NH₄)₂SO₄ 0.25%, NaCl 0.05%, MgSO₄·7H₂O 0.12%, K₂HPO₄·3H₂O 0.3%, CaCO₃ 0.3%, agar powder 1.5% %, the rest is distilled water and pH is 7.2 to 7.4.
Seed medium: corn starch 1.5%, glucose 1.0%, peanut meal 1.0%, soybean meal 0.8%, yeast powder 0.5%, CoCl₂·6H₂O 0.0003%, amylase 4u /g starch, the rest is tap water and pH is 7.0 to 7.2.
Fermentation medium: corn starch 6%, glucose 2%, peanut meal 1.4%, soybean meal 1.2%, yeast powder 0.3%, CoCl₂·6H₂O 0.0003%, amylase 4u/g starch, the rest is tap water and pH is 7.0 to 7.2

The AVE07-N2-16515 strain was inoculated on the plate medium, the culture temperature was 28 °C, and the culture time was 7 days; and then the plate-cultured strain was inoculated on the slant medium. After culturing at 28 °C for 7 days, the resulting of the slant culturing was inoculated into the seed medium for further culturing, in which a 250mL culture flask contains 30 mL of liquid and the culture temperature is 28 °C, rotation speed is 200 rpm and the culture time is 32 hours. Then, the seed culture solution was inoculated into the fermentation medium for further culturing, in which the initial pH is 7.2, the inoculum amount is 6% (v/v), the liquid volume in the 250mL culture flask is 50mL, rotation speed is 200 rpm and the culture temperature is 28.5 °C. After culturing for 9 days (216 hours) and testing, the enzyme activity of the fucoidan-degrading enzyme produced by fermentation was 303 U/mL, which is 22.9% higher than that of the original starting strain.

### Example 4:

This example illustrates the process for producing fucoidan-degrading enzyme by fermentation of the fucoidan-degrading enzyme-producing strain AVE07-N2-16515.

The composition of the medium used in this example:
Plate medium: soluble starch 1.0%, (NH₄)₂SO₄ 0.25%, NaCl 0.05%, MgSO₄·7H₂O 0.12%, K₂HPO₄·3H₂O 0.3%, CaCO₃ 0.3%, agar powder 1.5%, the rest is distilled water and pH is 7.2 to 7.4.
Slant medium: soluble starch 1.0%, (NH₄)₂SO₄ 0.25%, NaCl 0.05%, MgSO₄·7H₂O 0.12%, K₂HPO₄·3H₂O 0.3%, CaCO₃ 0.3%, agar powder 1.5% %, the rest is distilled water and pH is 7.2 to 7.4.
Seed medium: corn starch 1.5%, glucose 1.0%, peanut meal 1.0%, soybean meal 0.8%, yeast powder 0.5%, CoCl₂·6H₂O 0.0003%, amylase 4u /g starch, the rest is tap water and pH is 7.0 to 7.2.
Fermentation medium: corn starch 8%, peanut meal 1.4%, soybean meal 1.2%, yeast powder 0.3%, CoCl₂·6H₂O 0.0003%, amylase 4u/g starch, the rest is tap water and pH is 7.0 to 7.2.

The AVE07-N2-16515 strain was inoculated on the plate medium, the culture temperature was 28 °C, and the culture time was 7 days; and then the plate-cultured strain was inoculated on the slant medium. After culturing at 28 °C for 7 days, the resulting of the slant culturing was inoculated into the seed medium for further culturing, in which a 250mL culture flask contains 30 mL of liquid and the culture temperature is 28 °C, rotation speed is 200 rpm and the culture time is 32 hours. Then, the seed culture solution was inoculated into the fermentation medium for further culturing, in which the initial pH is 7.2, the inoculum amount is 6% (v/v), the liquid volume in the 250mL culture flask is 50mL, rotation speed is 200 rpm and the culture temperature is 28.5 °C. After culturing for 9 days (216 hours) and testing, the enzyme activity of the fucoidan-degrading enzyme produced by fermentation was 298 U/mL, which is 21.0% higher than that of the original starting strain.

### Example 5: This example illustrates the process for the production of fucoidan-degrading enzyme by fermentation of the fucoidan-degrading enzyme high-production strain AVE07-N2-16515.

The composition of the medium used in this example:
Plate medium: soluble starch 1.0%, (NH₄)₂SO₄ 0.25%, NaCl 0.05%, MgSO₄·7H₂O 0.12%, K₂HPO₄·3H₂O 0.3%, CaCO₃ 0.3%, agar powder 1.5%, the rest is distilled water and pH is 7.2 to 7.4.
Slant medium: soluble starch 1.0%, (NH₄)₂SO₄ 0.25%, NaCl 0.05%, MgSO₄·7H₂O 0.12%, K₂HPO₄·3H₂O 0.3%, CaCO₃ 0.3%, agar powder 1.5% %, the rest is distilled water and pH is 7.2 to 7.4.
Seed medium: corn starch 1.5%, glucose 1.0%, peanut meal 1.0%, soybean meal 0.8%, yeast powder 0.5%, CoCl₂·6H₂O 0.0003%, amylase 4u /g starch, the rest is tap water and pH is 7.0 to 7.2.
Fermentation medium: glucose 8%, peanut meal 1.4%, soybean meal 1.2%, yeast powder 0.3%, CoCl₂·6H₂O 0.0003%, amylase 4u/g starch, the rest is tap water and pH is 7.0 to 7.2.

The AVE07-N2-16515 strain was inoculated on the plate medium, the culture temperature was 28 °C, and the culture time was 7 days; and then the plate-cultured strain was inoculated on the slant medium. After culturing at 28 °C for 7 days, the resulting of the slant culturing was inoculated into the seed medium for further culturing, in which a 250mL culture flask contains 30 mL of liquid and the culture temperature is 28 °C, rotation speed is 200 rpm and the culture time is 32 hours. Then, the seed culture solution was inoculated into the fermentation medium for further culturing, in which the initial pH is 7.2, the inoculum amount is 6% (v/v), the liquid volume in the 250mL culture flask is 50mL, rotation speed is 200 rpm and the culture temperature is 28.5 °C. After culturing for 9 days (216 hours) and testing, the enzyme activity of the fucoidan-degrading enzyme produced by fermentation was 300 U/mL, which is 21.7% higher than that of the original starting strain.

### Example 6:

This example illustrates the process for the production of fucoidan-degrading enzyme by fermentation of the fucoidan-degrading enzyme high-production strain AVE07-N2-16515.

The composition of the medium used in this example:
Plate medium: soluble starch 1.0%, (NH₄)₂SO₄ 0.25%, NaCl 0.05%, MgSO₄·7H₂O 0.12%, K₂HPO₄·3H₂O 0.3%, CaCO₃ 0.3%, agar powder 1.5%, the rest is distilled water and pH is 7.2 to 7.4.
Slant medium: soluble starch 1.0%, (NH₄)₂SO₄ 0.25%, NaCl 0.05%, MgSO₄·7H₂O 0.12%, K₂HPO₄·3H₂O 0.3%, CaCO₃ 0.3%, agar powder 1.5% %, the rest is distilled water and pH is 7.2 to 7.4.
Seed medium: corn starch 1.5%, glucose 1.0%, peanut meal 1.0%, soybean meal 0.8%, yeast powder 0.5%, CoCl₂·6H₂O 0.0003%, amylase 4u /g starch, the rest is tap water and pH is 7.0 to 7.2.
Fermentation medium: maltose 8%, peanut meal 1.4%, soybean meal 1.2%, yeast powder 0.3%, CoCl₂·6H₂O 0.0003%, amylase 4u/g starch, the rest is tap water and pH is 7.0 to 7.2.

The AVE07-N2-16515 strain was inoculated on the plate medium, the culture temperature was 28 °C, and the culture time was 7 days; and then the plate-cultured strain was inoculated on the slant medium. After culturing at 28 °C for 7 days, the resulting of the slant culturing was inoculated into the seed medium for further culturing, in which a 250mL culture flask contains 30 mL of liquid and the culture temperature is 28 °C, rotation speed is 200 rpm and the culture time is 32 hours. Then, the seed culture solution was inoculated into the fermentation medium for further culturing, in which the initial pH is 7.2, the inoculum amount is 6% (v/v), the liquid volume in the 250mL culture flask is 50mL, rotation speed is 200 rpm and the culture temperature is 28.5 °C. After culturing for 9 days (216 hours) and testing, the enzyme activity of the fucoidan-degrading enzyme produced by fermentation was 389 U/mL, which is 17.3% higher than that of the original starting strain.

### Example 7:

This example illustrates the process for producing fucoidan degrading enzyme by fermentation of fucoidan degrading enzyme.

The composition of the medium used in this example:
Plate medium: soluble starch 1.0%, (NH₄)₂SO₄ 0.25%, NaCl 0.05%, MgSO₄·7H₂O 0.12%, K₂HPO₄·3H₂O 0.3%, CaCO₃ 0.3%, agar powder 1.5%, the rest is distilled water and pH is 7.2 to 7.4.
Slant medium: soluble starch 1.0%, (NH₄)₂SO₄ 0.25%, NaCl 0.05%, MgSO₄·7H₂O 0.12%, K₂HPO₄·3H₂O 0.3%, CaCO₃ 0.3%, agar powder 1.5% %, the rest is distilled water and pH is 7.2 to 7.4.
Seed medium: corn starch 1.5%, glucose 1.0%, peanut meal 1.0%, soybean meal 0.8%, yeast powder 0.5%, CoCl₂·6H₂O 0.0003%, amylase 4u /g starch, the rest is tap water and pH is 7.0 to 7.2.
Fermentation medium: corn starch 6%, glucose 2%, peanut meal 1.4%, soybean meal 1.2%, yeast powder 0.3%, CoCl₂·6H₂O 0.0003%, amylase 4u/g starch, the rest is tap water and pH is 7.0 to 7.2

The AVE07-N2-16515 strain was inoculated on the plate medium, the culture temperature was 28 °C, and the culture time was 7 days; and then the plate-cultured strain was inoculated on the slant medium. After culturing at 28 °C for 7 days, the resulting of the slant culturing was inoculated into the seed medium for further culturing, in which a 250mL culture flask contains 30 mL of liquid and the culture temperature is 28 °C, rotation speed is 200 rpm and the culture time is 32 hours. Then, the seed culture solution was inoculated into a 5L fermentation tank containing 3L fermentation medium for further culturing, in which the inoculum amount is 10% (v/v), rotation speed is 100 rpm and the culture temperature is 28.5 °C. After culturing for 216 hours and testing, the enzyme activity of the fucoidan-degrading enzyme produced by fermentation was 304 U/mL.

### Example 8:

The upstream and downstream homologous arms of the 16SDNA fragment of the target gene were fused and cloned into the PSKH plasmid by fusion PCR technology, and the recombinant plasmid was introduced into E. coli S17λpir, and then delivered into Bacillus subtilis strain by conjugation, the mutant strain was obtained by reverse screening of the sacB gene on the pDS132 plasmid.

The composition of medium fused for the mutant strain:
Plate medium: soluble starch 1.0%, (NH₄)₂SO₄ 0.25%, NaCl 0.05%, MgSO₄·7H₂O 0.12%, K₂HPO₄·3H₂O 0.3%, CaCO3 0.3%, agar powder 1.5%, the rest is distilled water and pH is 7.2 to 7.4.
Slant medium: soluble starch 1.0%, (NH₄)₂SO₄ 0.25%, NaCl 0.05%, MgSO₄·7H₂O 0.12%, K₂HPO₄·3H₂O 0.3%, CaCO₃ 0.3%, agar powder 1.5% %, the rest is distilled water and pH is 7.2 to 7.4.
Seed medium: corn starch 1.5%, glucose 1.0%, peanut meal 1.0%, soybean meal 0.8%, yeast powder 0.5%, CoCl₂·6H₂O 0.0003%, amylase 4u /g starch, the rest is tap water and pH is 7.0 to 7.2.
Fermentation medium: corn starch 6%, glucose 2%, peanut meal 1.4%, soybean meal 1.2%, yeast powder 0.3%, CoCl₂·6H₂O 0.0003%, amylase 4u/g starch, the rest is tap water and pH is 7.0 to 7.2

The AVE07-N2-16515 strain was inoculated on the plate medium, the culture temperature was 28 °C, and the culture time was 7 days; and then the plate-cultured strain was inoculated on the slant medium. After culturing at 28 °C for 7 days, the resulting of the slant culturing was inoculated into the seed medium for further culturing, in which a 250mL culture flask contains 30 mL of liquid and the culture temperature is 28 °C, rotation speed is 200 rpm and the culture time is 32 hours. Then, the seed culture solution was inoculated into a 5L fermentation tank containing 3L fermentation medium for further culturing, in which the inoculum amount is 10% (v/v), rotation speed is 100 rpm and the culture temperature is 28.5 °C. After culturing for 216 hours and testing, the enzyme activity of the fucoidan-degrading enzyme produced by fermentation was 610 U/mL.

Compared with the example, the process conditions are completely consistent, while the 16S DNA fragment of the target gene is repeatedly cloned by the fusion PCR technology, and the enzyme activity is doubled, so that it can be determined that the gene fragment is an active fragment, which plays a key role.

The above are only the preferred embodiments of the present invention. It should be pointed out that for those of ordinary skill in the art, without departing from the principle of the present invention, improvements and modifications can be made. These improvements and modifications should also be regarded as falling in the protection scope of the present invention.

## Claims

1. 16s rDNA, wherein its sequence is as shown in SEQ ID NO:1.

2. The use of the 16S rDNA of claim 1 in the preparation of fucoidan degrading enzyme-producing genetic engineered strains.

3. A plasmid, wherein it comprises the 16S rDNA of claim 1.

4. The plasmid according to claim 3, wherein the vector of the plasmid is a PSKH plasmid.

5. A genetic engineered strain, wherein it comprises 16S rDNA of claim 1 or the plasmid of claim 3 or 4.

6. A fermentation method of the genetically genetic engineered strain according to claim 5, wherein it comprises the following steps:
(1) Plate culturing: inoculating the strain on a plate medium, the culturing temperature is 26-30 °C, and the culturing time is 6 ^{~}7d;
(2) Slant culturing: inoculating the strain cultured in step (1) on a slant medium, the culturing temperature is 26-30 °C, and the culturing time is 6-7d;
(3) Seed culturing: inoculating the strain cultured in step (2) into a seed medium, the liquid volume is 8vt%-20vt%, the culturing temperature is 26-30 °C, and the rotation speed is 180-220rpm, and the culturing time is 30-45h;
(4) Fermentation culturing: inoculating the seed culture solution in step (3) into the fermentation medium, the inoculum is 2%-10% (v/v), the liquid volume is 14vt%-26vt%, the culturing temperature is 26-30 °C, and culturing for 8-10 days at180-240rpm rotation speed.

7. The fermentation method according to claim 6, wherein
- the plate medium contains the following components in mass percentage: carbon source 0.5% to 1.5%, nitrogen source 0.1% to 0.1%. 3%, inorganic salt 0.5% to 1.0%, agar 1.2% to 1.8%, the rest is water, pH is 7.2 to 7.4;
- in the plate medium, the carbon source is one of or a mixture of glucose and soluble starches; the nitrogen source is one of or a mixture of ammonium acetate and ammonium sulfate; the inorganic salt is one of or a mixture of two or more of sodium salt, potassium salt, magnesium salt, and calcium salt.

8. The fermentation method according to claim 6, wherein
- the slant medium contains the following components in mass percentage: carbon source 0.5% to 1.5%, nitrogen source 0.1% to 0.1%. 3%, inorganic salt 0.5% to 1.0%, agar 1.2% to 1.8%, the rest is water, pH is 7.2 to 7.4;
- in the slant medium, the carbon source is one of or a mixture of glucose and soluble starches; the nitrogen source is one of or a mixture of ammonium acetate and ammonium sulfate; the inorganic salt is one of or a mixture of two or more of sodium salt, potassium salt, magnesium salt, and calcium salt.

9. The fermentation method according to claim 6, wherein
- the seed medium contains the following components in mass percentage: carbon source 2.0% to 3.0%, nitrogen source 2.0% to 4.0%, trace elements 0.0001% to 0.0004%, amylase 3 to 5 u/g starch, the rest is water, pH is 7.0 to 7.2;
- in the seed culture medium, the carbon source is one of or a mixture of corn starch and glucose; the nitrogen source is one of or a mixture of two or more of peanut meal, soybean meal, corn steep liquor, yeast powder and yeast extract; and the trace element is cobalt chloride.

10. The fermentation method according to any one of claims 6 to 9, wherein
- the fermentation medium contains the following components in mass percentage: carbon source 5.0% to 15.0%, nitrogen source 2.0%^{~}4.0%, trace element 0.0001%^{~}0.0004%, amylase 3^{~}5u/g starch, the rest is water, pH is 7.0^{~}7.2;
- in the fermentation medium, the carbon source is one of or a mixture of two or more of corn starch, glucose and maltose; the nitrogen source is one of or a mixture of two or more of peanut meal, soybean meal, corn steep liquor and yeast powder; the trace element is cobalt chloride.


SEQUENCE LISTING

<110>  Beijing Leili Marine Bioindustry Inc.

<120>  FUCOIDAN DEGRADING ENZYME-PRODUCING GENETIC ENGINEERED STRAIN AND
PREPARATION METHOD THEREOF

<130>  0653PA21001EP

<150>  CN202011558474X
<151>  2020-12-24

<160>  1

<170>  SIPOSequenceListing 1 .0

<210>  1
<211>  1453
<212>  DNA
<213>  Artificial Sequence

<400>  1
gcagctacag atagacatga tcgtcgagca gagtggatta agagctgtgc tcttatgaag      60

ttagcggcgg acgggtgagt aacacgtggg taacctgccc ataagactgg gataactccg     120

ggaaaccggg gctaataccg gataacattt tgaaccgcat ggttcgaaat tgaaaggcgg     180

cttcggctgt cacttatgga tggacccgcg tcgcattagc tagttggtga ggtaacggct     240

caccaaggca acgatgcgta gccgacctga gagggtgatc ggccacactg ggactgagac     300

acggcccaga ctcctacggg aggcagcagt agggaatctt ccgcaatgga cgaaagtctg     360

acggagcaac gccgcgtgag tgatgaaggc tttcgggtcg taaaactctg ttgttaggga     420

agaacaagtg ctagttgaat aagctggcac cttgacggta cctaaccaga aagccacggc     480

taactacgtg ccagcagccg cggtaatacg taggtggcaa gcgttatccg gaattattgg     540

gcgtaaagcg cgcgcaggtg gtttcttaag tctgatgtga aagcccacgg ctcaaccgtg     600

gagggtcatt ggaaactggg agacttgagt gcagaagagg aaagtggaat tccatgtgta     660

gcggtgaaat gcgtagagat atggaggaac accagtggcg aaggcgactt tctggtctgt     720

aactgacact gaggcgcgaa agcgtgggga gcaaacagga ttagataccc tggtagtcca     780

cgccgtaaac gatgagtgct aagtgttaga gggtttccgc cctttagtgc tgaagttaac     840

gcattaagca ctccgcctgg ggagtacggc cgcaaggctg aaactcaaag gaattgacgg     900

gggcccgcac aagcggtgga gcatgtggtt taattcgaag caacgcgaag aaccttacca     960

ggtcttgaca tcctttgaca accctagaga tagggcttct ccttcgggag cagagtgaca    1020

ggtggtgcat ggttgtcgtc agctcgtgtc gtgagatgtt gggttaagtc ccgcaacgag    1080

cgcaaccctt gattttagtt gccatcattt agttgggcac tttaaggtga ctgccggtga    1140

caaaccggag gaaggtgggg atgacgtcaa atcatcatgc cccttatgac ctgggctaca    1200

cacgtgctac aatggacggt acaaagagct gcaagaccgc gaggtggagc taatctcata    1260

aaaccgttct cagttcggat tgtaggctgc aactcgccta catgaagctg gaatcgctag 1320

taatcgcgga tcagcatgcc gcggtgaata cgttcccggg ccttgtacac accgcccgtc 1380

acaccacgag agtttgtaac accgagaagt cggtggggta accaagtgga gccaacgtac 1440

ctaaagagct gcg 1453